# EUROPÄISCHE PATENTSCHRIFT

(11) **EP 1 514 955 B1**
(45) Veröffentlichungstag und Bekanntmachung des Hinweises auf die Patenterteilung: **31.05.2006**
(21) Anmeldenummer: 04021038.7
(22) Anmeldetag: 03.09.2004
(51) Int. Cl.: C25B 15/08, C25B 3/02, C07C 43/32

(54) **Verfahren zur destillativen Aufarbeitung eines Tmof enthaltenden Elektrolyseaustrages**
Method for distilling an electrolyte containing tmof
Procédé de distillation d'un electrolyte contenant tmof

(30) Priorität: 04.09.2003 DE 10340737
(43) Veröffentlichungstag der Anmeldung: 16.03.2005
(73) Patentinhaber: BASF Aktiengesellschaft, 67056 Ludwigshafen (DE)
(72) Erfinder: Heydrich, Gunnar, Dr., 67117 Limburgerhof (DE); Richter, Ingo, Dr., 68165 Mannheim (DE); Krug, Thomas, 67550 Worms (DE)
(74) Vertreter: Isenbruck, Günter

(56) Entgegenhaltungen:
- EP-A- 1 002 783
- EP-A- 1 151 781
- WO-A-02/20446
- DE-A- 10 209 195

## Beschreibung

Die Erfindung betrifft ein Verfahren zur destillativen Aufarbeitung des Elektrolyseaustrages der elektrochemischen Oxidation von 1,1,2,2-Tetramethoxyethan (TME) mit Methanol zu Trimethylorthoformiat (TMOF) in einem flüssigen Elektrolyten.

TMOF ist ein bedeutendes Zwischenprodukt für die Herstellung von Wirkstoffen.

Neben dem klassischen Verfahren, durch Umsetzung von Chloroform mit Natriummethylat, gewinnt die elektrochemische oxidative Spaltung von Diacetalen des Glyoxals zunehmend an Bedeutung.

Es ist allgemein bekannt, dass Diacetale des Glyoxals durch sauer katalysierte Acetalisierung von Glyoxal mit einwertigen Alkoholen R-OH hergestellt werden können:
Die säurekatalysierte Acetalisierung von Glyoxal mit einwertigen Alkoholen ist eine komplexe Reaktion, bei der neben dem Monoacetal und dem Diacetal auch eine Vielzahl von Oligomeren und/oder cyclischen Nebenprodukten gebildet werden können (siehe zum Beispiel J.M. Kliegmann et al. in J. Org. Chem., Vol. 38 (1973), S. 556).

Die vorliegende Erfindung ist nicht auf ein spezielles Verfahren zur Herstellung des Diacetals des Glyoxals mit Methanol, dem 1,1,2,2-Tetramethoxyethan (TME), eingeschränkt, sondern grundsätzlich auf jedes mögliche Herstellungsverfahren für TME anwendbar.

Besonders vorteilhaft kann TME durch Umsetzung von Glyoxal mit Methanol in Gegenwart eines sauren Katalysators hergestellt werden, indem man Glyoxal und Methanol mit dem sauren Katalysator so lange in Kontakt belässt, bis die Konzentration des gebildeten Diacetals in der Reaktionsmischung wenigstens 70 % der Gleichgewichtskonzentration erreicht hat. Ein derartiges Verfahren ist in der nicht vorveröffentlichten deutschen Patentanmeldung 102 09 195 beschrieben.

Das TME wird anschließend durch elektrochemische Oxidation mit Methanol zum TMOF umgesetzt. Hierbei wird ein Elektrolyseaustrag erhalten, der anschließend, insbesondere zur Abtrennung von reinem TMOF, destillativ aufgearbeitet wird.
Für die kontinuierliche destillative Zerlegung von Mehrstoffgemischen sind verschiedene Verfahrensvarianten gebräuchlich. Im einfachsten Fall wird das Zulaufgemisch in zwei Fraktionen, eine leichtsiedende Kopffraktion und eine schwersiedende Sumpffraktion, zerlegt. Bei der Auftrennung von Zulaufgemischen in mehr als zwei Fraktionen müssen nach dieser Verfahrensvariante mehrere Destillationskolonnen eingesetzt werden. Um den apparativen Aufwand zu begrenzen, setzt man bei der Auftrennung von Vielstoffgemischen nach Möglichkeit Kolonnen mit flüssigen oder dampfförmigen Seitenabzügen ein. Die Anwendungsmöglichkeit von Destillationskolonnen mit Seitenabzügen ist jedoch dadurch stark eingeschränkt, dass die an den Seitenabzugsstellen entnommenen Produkte nie völlig rein sind. Bei Seitenentnahmen im Verstärkungsteil, die üblicherweise in flüssiger Form erfolgen, enthält das Seitenprodukt noch Anteile an leichtsiedenden Komponenten, die über Kopf abgetrennt werden sollen. Entsprechendes gilt für Seitenentnahmen im Abtriebsteil, die meist dampfförmig erfolgen, bei denen das Seitenprodukt noch Hochsiederanteile aufweist. Die Verwendung von konventionellen Seitenabzugskolonnen ist daher auf Fälle begrenzt, in denen verunreinigte Seitenprodukte zulässig sind.

Eine Abhilfemöglichkeit bieten sogenannte Trennwandkolonnen, die beispielsweise in EP-A 0 122 367 beschrieben sind. Trennwandkolonnen sind Destillationskolonnen mit senkrechten Trennwänden, die in Teilbereichen eine Quervermischung von Flüssigkeits- und Brüdenströmen verhindern. Die Trennwand, die vorzugsweise aus einem Blech besteht, unterteilt die Kolonne in Längsrichtung in deren mittlerem Bereich in einen Zulaufteil und in einen Entnahmeteil.

Aus Trennwandkolonnen können, neben einem Kopf- und einem Sumpfprodukt, Seitenprodukte ebenfalls in reiner Form erhalten werden.

Ein ähnliches Ergebnis kann mit sogenannten thermisch gekoppelten Kolonnen erreicht werden, d.h. mit Anordnungen von mindestens zwei Kolonnen, wobei jede der Kolonnen mit jeder anderen mindestens zwei Verknüpfungen an räumlich getrennten Stellen aufweist.

Trennwandkolonnen und thermisch gekoppelte Kolonnen haben gegenüber Anordnungen von konventionellen Destillationskolonnen niedrigere Investitions- und Energiekosten.

Es war Aufgabe der Erfindung, ein verbessertes, insbesondere wirtschaftlicheres Verfahren zur destillativen Aufarbeitung des Elektrolyseaustrages der elektrochemischen Oxidation von TME mit Methanol zu TMOF in einem flüssigen Elektrolyten zur Verfügung zu stellen.

Die Lösung besteht in einem Verfahren zur destillativen Aufarbeitung des Elektrolyseaustrages a der elektrochemischen Oxidation von 1,1,2,2-Tetramethoxyethan (TME) mit Methanol zu Trimethylorthoformiat (TMOF) in einem flüssigen Elektrolyten, dadurch gekennzeichnet, dass man die destillative Aufarbeitung in einer Trennwandkolonne (TK) mit 30 bis 150 theoretischen Trennstufen durchführt, in der eine Trennwand (T) in Kolonnenlängsrichtung und Ausbildung eines oberen gemeinsamen Kolonnenbereiches, eines unteren gemeinsamen Kolonnenbereiches, eines Zulaufteils mit Verstärkungsteil und Abtreibsteil sowie eines Entnahmeteils mit Abtriebsteil und Verstärkungsteil angeordnet ist, mit Zuführung des Elektrolyseaustrags a in den mittleren Bereich des Zulaufteils, Gewinnung von TMOF als Seitenabzug aus dem mittleren Bereich des Entnahmeteils und Abführung einer oder mehrerer Leichtsiederfraktionen aus dem oberen gemeinsamen Kolonnenbereich und ein oder mehrerer Hochsiederfraktionen aus dem unteren gemeinsamen Kolonnenbereich.

Es wurde überraschend gefunden, dass die anspruchsvolle destillative Trennaufgabe zur Auftrennung des Elektrolyseaustrages der elektrochemischen Oxidation von TME mit Methanol zu TMOF, der ein komplexes Gemisch ist, auch in den gegenüber klassischen Destillationskolonnen bekanntermaßen schwieriger zu beherrschenden Trennwandkolonnen beziehungsweise thermisch gekoppelten Kolonnen, mit entsprechenden wirtschaftlichen Vorteilen, erfolgreich gelöst werden kann.

Die elektrochemische Oxidation von TME mit Methanol zu TMOF kann beispielsweise so durchgeführt werden, wie in den deutschen Patentanmeldungen 100 43 789, 100 58 304 oder 101 46 566 beschrieben. Bevorzugt wird die elektrochemische Oxidation wie in der nicht vorveröffentlichten deutschen Patentanmeldung 102 09 195 beschrieben durchgeführt.

Bevorzugt wird das molare Verhältnis aus der Summe der Menge an TME und TMOF zur Menge an Methanol im Elektrolyten, in dem die elektrochemische Oxidation durchgeführt wird, im Bereich von 0,2 : 1 bis 5 : 1, bevorzugt im Bereich von 0,2 : 1 bis 2 : 1 und besonders bevorzugt im Bereich von 0,3 : 1 bis 1: 1, eingestellt.

Als Leitsalze, die in der Elektrolyselösung enthalten sind, handelt es sich im Allgemeinen um Alkali, Tetra(C₁- bis C₆-alkyl)ammonium- oder Tri(C₁- bis C₆-alkyl) benzylammoniumsalze. Als Gegenion kommen Sulfat, Hydrogensulfat, Alkylsulfate, Arylsulfate, Halogenide, Phosphate, Carbonate, Alkylphosphate, Alkylcarbonate, Nitrat, Alkoholate, Tetrafluorborat oder Perchlorat in Betracht.

Weiterhin kommen die von den vorstehend genannten Anionen abgeleiteten Säuren als Leitsalze in Betracht.

Bevorzugt sind Methyltributylammoniummethylsulfat (MTBS), Methyltriethylammoniummethylsulfat oder Methyl-tri-propylmethylammoniummethylsulfat.

Gegebenenfalls setzt man der Elektrolyselösung übliche Cosolvenzien zu. Dabei handelt es sich um die in der organischen Chemie allgemein üblichen inerten Lösungsmittel mit einem hohen Oxidationspotential. Beispielhaft genannt seien Dimethylcarbonat oder Propylencarbonat.

An der Kathode können verschiedenartige elektrochemische Reduktionen an organischen Verbindungen durchgeführt werden. Solche sind insbesondere in der DE-A 100 58 304 beschrieben. Im Allgemeinen wird jedoch an der Kathode durch elektrochemische Reduktion von Protonen oder Alkohol Wasserstoff entwickelt.

Die elektrochemische Umsetzung kann in allen üblichen Elektrolysezellentypen durchgeführt werden. Vorzugsweise arbeitet man kontinuierlich mit ungeteilten Durchflusszellen. Besonders bevorzugt sind kontinuierlich durchströmte bipolar geschaltete Plattenstapelzellen, wie sie beispielsweise in der DE-A 195 33 773 beschrieben sind.

Die Stromdichten, bei denen man das Verfahren durchführt, betragen im Allgemeinen 1 bis 1000, bevorzugt 10 bis 100 mA/cm². Die Temperaturen betragen üblicherweise -20 bis 60°C, bevorzugt 0 bis 60 °C. Im Allgemeinen wird bei Normaldruck gearbeitet. Höhere Drücke werden bevorzugt dann angewandt, wenn bei höheren Temperaturen gearbeitet werden soll, um ein Sieden der Ausgangsverbindungen beziehungsweise Cosolventien zu vermeiden.

Als Anodenmaterialien eignen sich beispielsweise Edelmetalle wie Platin oder Metalloxide wie Ruthenium- oder Chromoxid oder Mischoxide des Typs RuOₓTiOₓ. Bevorzugt sind Graphit oder Kohleelektroden.

Als Kathodenmaterialien kommen beispielsweise Eisen, Stahl, Edelstahl, Nickel oder Edelmetalle wie Platin sowie Graphit oder Kohlematerialien in Betracht. Bevorzugt ist das System Graphit als Anode und Kathode sowie Graphit als Anode und Nickel, Edelstahl oder Stahl als Kathode.

Besonders bewährt hat sich eine Verfahrensvariante, bei der als Elektrolyt, der einer kontinuierlich durchströmten Plattenstapelzelle zugeführt wird, eine Mischung enthaltend im Wesentlichen 50 bis 75, bevorzugt 63 Gew.-% TME, 15 bis 40, bevorzugt 27 Gew.-% Methanol und 1 bis 20, bevorzugt 10 Gew.-% MTBS eingesetzt wird.

Die Strömungsgeschwindigkeit und Stromdichte wird bevorzugt so eingestellt, dass der Elektrolyt, der der Plattenstapelzelle entnommen wird, folgende Zusammensetzung aufweist: 25 bis 50, bevorzugt 40 Gew.-% TME, 25 bis 45, bevorzugt 34 Gew.-% TMOF, 5 bis 30, bevorzugt 14 Gew.-% Methanol und 1 bis 20, bevorzugt 10 Gew.-% MTBS sowie je 0,5 bis 2, bevorzugt 1 Gew.-% Methylformiat und Methylal.

Der Elektrolyseaustrag wird erfindungsgemäß in einer Trennwandkolonne oder in thermisch gekoppelten Kolonnen aufgetrennt. Trennwandkolonnen weisen typischerweise eine in Kolonnenlängsrichtung ausgerichtete Trennwand auf, die den Kolonneninnenraum in die folgenden Teilbereiche unterteilt: einen oberen gemeinsamen Kolonnenbereich, einen unteren gemeinsamen Kolonnenbereich sowie einen Zulaufteil und einen Entnahmeteil, jeweils mit Verstärkungsteil und Abtriebsteil. Das aufzutrennende Gemisch wird im Bereich des Zulaufteils aufgegeben, eine oder mehrere Hochsiederfraktionen werden aus dem Kolonnensumpf, eine oder mehrere Leichtsiederfraktionen über den Kolonnenkopf und eine oder mehrere Mittelsiederfraktionen aus dem Bereich des Entnahmeteils entnommen.

Bevorzugt führt man die destillative Auftrennung in einer Trennwandkolonne mit 60 bis 90 theoretischen Trennstufen und bei einem Betriebsdruck zwischen 300 und 1500 mbar, insbesondere zwischen 400 und 600 mbar, durch.

Vorteilhaft sind die theoretischen Trennstufen der Trennwandkolonne dergestalt auf die einzelnen Kolonnenbereiche 1 bis 6 aufgeteilt, dass dieselben jeweils 5 bis 50 %, bevorzugt jeweils 15 bis 30 % der Gesamtzahl der theoretischen Trennstufen der Trennwandkolonne aufweisen.

Bevorzugt kann der Elektrolyseaustrag vor der Zuführung zur Trennwandkolonne einer ein- oder mehrstufigen Vorverdampfung unterworfen werden und anschließend zweiphasig oder in Form von zwei Strömen, eines gasförmigen und eines flüssiges Stromes, der Trennwandkolonne zugeführt werden. Die Vorverdampfung bietet sich insbesondere dann an, wenn der Elektrolyseaustrag größere Mengen an Leichtsiedern enthält. Durch die Vorverdampfung kann der Abtriebsteil der Trennwandkolonne wesentlich entlastet werden.

Vorliegend werden als Hochsieder Substanzen bezeichnet, deren Siedepunkt oberhalb des Siedepunkts des Wertproduktes TMOF, d.h. oberhalb von 101 bis 102 °C bei Normaldruck liegt, beziehungsweise als Leichtsieder Substanzen, deren Siedepunkt unterhalb des Siedepunktes von TMOF liegt.

In einer vorteilhaften Verfahrensvariante wird, zusätzlich zur Sumpfflüssigkeit eine Hochsiederfraktion aus dem unteren gemeinsamen Kolonnenbereich von einer Trennstufe, die oberhalb des Kolonnensumpfes liegt, bevorzugt von der ersten bis 20. insbesondere von der ersten bis vierten theoretischen Trennstufe, bei üblicher Zählung der theoretischen Trennstufen von unten nach oben in der Kolonne und teilweise oder vollständig ausgeschleust. Durch diese Fahrweise kann insbesondere die spezifikationsschädliche Nebenkomponente 2-Methoxy-1,3-dioxolan aus dem Verfahren ausgeschleust werden, die dann in der Elektrolyse gebildet werden kann, wenn das als Edukt eingesetzte TME als Verunreinigung 2,3-Dimethyoxy-1,4-diöxan enthält.

Vorteilhaft kann aus dem oberen gemeinsamen Kolonnenbereich unterhalb des Kolonnenkopfes, bevorzugt von der dritten bis sechsten theoretischen Trennstufe unterhalb des Kolonnenkopfes, eine Leichtsiederfraktion als flüssiger Seitenabzug gewonnen werden, die überwiegend Methanol enthält, bevorzugt oberhalb von 90 Gew.-%, insbesondere oberhalb 97 Gew.-% Methanol und die vorteilhaft in die Reaktionsstufe recycliert wird. Die Abnahme dieser Leichtsiederfraktion wird bevorzugt über eine Temperaturregelung gesteuert: Bei steigender Temperatur wird die Abnahmemenge gedrosselt und so ein höherer interner Rücklauf erzeugt, der wiederum zum Sinken der Temperatur führt.

Bei der Trennung von Mehrstoffgemischen in eine Leichtsieder-, eine Mittelsieder- und eine Hochsiederfraktion werden üblicherweise Spezifikationen über den maximal zulässigen Anteil an Leichtsiedern und Hochsiedern in der Mittelsiederfraktion vorgegeben.

Hierbei werden für das Trennproblem kritische Komponenten, sogenannte Schlüsselkomponenten oder die Summe von mehreren Schlüsselkomponenten spezifiziert.

Im vorliegenden Verfahren sind Schlüsselkomponenten Methanol als Leichtsieder und TME beziehungsweise 2-Methoxy-1,3-dioxolan, wenn im Kolonnenzulauf vorhanden, als Hochsieder.

Die Einhaltung der Spezifikation für die Hochsieder in der Mittelsiederfraktion wird über das Aufteilungsverhältnis der Flüssigkeit am oberen Ende der Trennwand gewährleistet. Dieses wird bevorzugt so eingestellt, dass die Konzentration der hochsiedenden Schlüsselkomponenten in der Flüssigkeit am oberen Ende der Trennwand 10 bis 80 %, bevorzugt 30 bis 50 % des Wertes ausmacht, der im Seitenabzugsprodukt erzielt werden soll. Die Flüssigkeitsaufteilung wird so eingestellt, dass bei höheren Gehalten an hochsiedenden Schlüsselkomponenten mehr und bei niedrigeren Gehalten an hochsiedenden Schlüsselkomponenten weniger Flüssigkeit auf den Zulaufteil geleitet wird.

Entsprechend wird die Spezifikation für den Leichtsieder in der Mittelsiederfraktion durch die Heizleistung des Sumpfverdampfers geregelt. Hierbei wird die Heizleistung des Sumpfverdampfers bevorzugt so eingestellt, dass die Konzentration der leichtsiedenden Schlüsselkomponenten in der Flüssigkeit am unteren Ende der Trennwand 10 bis 80%, bevorzugt 30 bis 50% des Wertes beträgt, der im Seitenabzugsprodukt erzielt werden soll und die Heizleistung dahingehend eingestellt wird, dass bei höheren Gehalten an leichtsiedenden Schlüsselkomponenten die Heizleistung erhöht und bei niedrigeren Gehalten an leichtsiedenden Schlüsselkomponenten die Heizleistung verringert wird.

Zur Kompensation von Störungen der Zulaufmenge oder der Zulaufkonzentration erwies es sich als vorteilhaft, über entsprechende Regelvorschriften im Prozessleitsystem sicherzustellen, dass die Mengenströme der Flüssigkeiten, die auf den Verstärkungsteil des Zulaufteils oder auf den Verstärkungsteil des Entnahmeteils aufgegeben werden, nicht unter 30% ihres Normalwertes sinken können.

Hierzu wird die Flüssigkeit über eine Pumpe gefördert oder über eine statische Zulaufhöhe von mindestens 1 m mengengeregelt aufgegeben, bevorzugt über eine Kaskadenregelung in Verbindung mit der Flüssigkeitsstandregelung des Auffangraumes, wie nachfolgend beschrieben.

Zur Entnahme und Aufteilung der Flüssigkeiten am oberen Ende der Trennwand und an der Seitenentnahmestelle eignen sich sowohl innenliegende als auch außerhalb der Kolonne angeordnete Auffangräume für die Flüssigkeit, die die funktionelle Pumpenvorlage übernehmen oder für eine ausreichend hohe statische Flüssigkeitshöhe sorgen, und eine durch Stellorgane, beispielsweise Ventile, geregelte Flüssigkeitsweiterleitung ermöglichen. Bei der Verwendung von gepackten Trennwandkolonnen wird die Flüssigkeit zunächst in Sammlern gefasst und von dort aus in einen innenliegenden oder außenliegenden Auffangraum geleitet.

Bezüglich der trennwirksamen Einbauten können in den gemeinsamen sowie auch in den durch die Trennwand unterteilten Teilbereichen Böden, Füllkörper oder bevorzugt geordnete Packungen eingesetzt werden. Geordnete Blech- oder Gewebepackungen mit spezifischen Oberflächen im Bereich von 100 bis 1000 m²/m³, insbesondere im Bereich von etwa 350 bis 500 m²/m³ sind besonders geeignet.

Eine besonders hohe Produktreinheit kann erreicht werden, wenn die Trennwand wärmeisolierend ausgeführt ist. Besonders vorteilhaft ist eine doppelwandige Ausführung mit dazwischenliegendem engem Gasraum.

Die Mittelsiederfraktion kann als flüssiger oder gasförmiger Seitenabzug aus dem Entnahmeteil der Trennwandkolonne abgezogen werden. Bevorzugt wird die Mittelsiederfraktion an der Seitenabzugsstelle in flüssiger Form entnommen.

In einer bevorzugten Ausführungsvariante sind die Zuführung und der Seitenabzug aus der Trennwandkolonne auf gleicher Höhe in der Trennwandkolonne angeordnet.

In einer weiteren bevorzugten Ausführungsvariante sind die Zuführung und der Seitenabzug aus der Trennwandkolonne auf unterschiedlicher Höhe in der Trennwandkolonne angeordnet, und zwar um 1 bis 20 theoretische Trennstufen, bevorzugt um 5 bis 10 theoretische Trennstufen, wobei der Seitenabzug bevorzugt höher gegenüber der Zuführung angeordnet ist.

Die Verteilung des Brüdenstromes am unteren Ende der Trennwand kann durch die Wahl und/oder Dimensionierung der Trenneinbauten und/oder den Einbau Druckverlust erzeugender Einrichtungen, beispielsweise von Blenden, so eingestellt werden, dass das Verhältnis des Brüdenstromes im Zulaufteil zu dem des Entnahmeteils im Bereich von 0,5 bis 2,0 bevorzugt im Bereich von 0,9 bis 1,1, liegt.

Vorteilhaft wird die aus dem oberen gemeinsamen Kolonnenbereich ablaufende Flüssigkeit in einem innerhalb oder außerhalb der Trennwandkolonne angeordneten Auffangraum gesammelt und durch eine Festeinstellung oder Regelung am oberen Ende der Trennwand so aufgeteilt, dass das Verhältnis des Flüssigkeitsstromes zum Zulaufteil zum Flüssigkeitsstrom zum Entnahmeteil im Bereich von 0,1 bis 2,5, bevorzugt im Bereich von 0,4 bis 0,8, bei überwiegend flüssigem Zulauf und bevorzugt im Bereich von 1,0 bis 2,0 bei gasförmigem Zulauf zur Trennwandkolonne, beträgt.

Vorteilhaft wird die Flüssigkeit auf den Zulaufteil über eine Pumpe gefördert oder über eine statische Zulaufhöhe von mindestens 1 m mengengeregelt aufgegeben, bevorzugt über eine Kaskadenregelung in Verbindung mit der Flüssigkeitsstandregelung des Auffangraumes, wobei die Regelung so eingestellt wird, dass die auf den Zulaufteil aufgegebene Flüssigkeitsmenge nicht unter 30 % ihres Normalwertes sinken kann.

Vorteilhaft wird die Aufteilung der aus dem Abtriebsteil des Entnahmeteils der Kolonne ablaufende Flüssigkeit auf den Seitenabzug und den Verstärkungsteil des Entnahmeteils durch eine Regelung so eingestellt, dass die auf den Verstärkungsteil des Entnahmeteils aufgegebene Flüssigkeitsmenge nicht unter 30 % ihres Normalwertes sinken kann.

Die Trennwandkolonne ist vorteilhaft am oberen und am unteren Ende der Trennwand mit Probenahmemöglichkeiten bestückt, aus der aus der Kolonne kontinuierlich oder in zeitlichen Abständen flüssige und/oder gasförmige Proben entnommen und hinsichtlich ihrer Zusammensetzung, bevorzugt gaschromatographisch untersucht werden.

Die Destillatentnahme erfolgt bevorzugt temperaturgeregelt, wobei als Regeltemperatur eine Messstelle im oberen gemeinsamen Kolonnenbereich verwendet wird, die bevorzugt um 3 bis 8 theoretische Trennstufen, insbesondere um 4 bis 6 theoretische Trennstufen vom oberen Ende der Trennwandkolonne entfernt ist.

Die Entnahme des Sumpfproduktes erfolgt bevorzugt ebenfalls temperaturgeregelt, wobei als Regeltemperatur eine Messstelle im unteren gemeinsamen Kolonnenbereich verwendet wird, die um 3 bis 8, insbesondere um 4 bis 6 theoretische Trennstufen oberhalb des unteren Endes der Kolonne angeordnet ist.

Die Entnahme des Seitenproduktes erfolgt bevorzugt standgeregelt, wobei als Regelgröße der Flüssigkeitsstand im Kolonnensumpf verwendet wird.

Bevorzugt kann die Flüssigkeitsverteilung auf die einzelnen Teilbereiche der Kolonne gezielt ungleichmäßig eingestellt werden, wobei insbesondere die Flüssigkeit in den Verstärkungsteilen von Zulauf- und Entnahmeteil verstärkt im Wandbereich und in den Abtriebsteilen von Zulauf- und Entnahmeteil reduziert im Wandbereich aufgegeben wird.

Als Sumpfverdampfer für die Trennwandkolonne kann vorteilhaft ein Dünnschichtapparat, beispielsweise ein Fallfilmverdampfer, eingesetzt werden.

Die Trennwand kann in der Kolonne fest eingeschweißt, jedoch auch in Form von lose gesteckten und adäquat abgedichteten Teilsegmenten ausgestaltet sein.

Erfindungsgemäß ist es auch möglich, anstelle der Trennwandkolonne thermisch gekoppelte Kolonnen einzusetzen. Anordnungen mit thermisch gekoppelten Kolonnen sind hinsichtlich des Energiebedarfs mit einer Trennwandkolonne gleichwertig. Diese Erfindungsvariante bietet sich insbesondere bei Verfügbarkeit von bestehenden Kolonnen an. Die geeignetsten Formen der Zusammenschaltung können je nach Trennstufenzahl der vorhandenen Kolonnen ausgewählt werden.

Die thermisch gekoppelten Kolonnen können jeweils mit einem eigenen Verdampfer und/oder Kondensator ausgestattet sein.

In einer bevorzugten Verfahrensvariante werden in den Verbindungsströmen zwischen den beiden thermisch gekoppelten Kolonnen nur Flüssigkeiten gefördert. Dies ist besonders vorteilhaft, wenn die thermisch gekoppelten Kolonnen mit unterschiedlichen Drücken betrieben werden.

In einer bevorzugten Verschaltung der thermisch gekoppelten Kolonnen werden die Leichtsiederfraktion und die Hochsiederfraktion aus unterschiedlichen Kolonnen entnommen, wobei der Betriebsdruck der Kolonne, aus der die Hochsiederfraktion entnommen wird, tiefer eingestellt wird als der Betriebsdruck der Kolonne, aus der die Leichtsiederfraktion entnommen wird, bevorzugt um 0,5 bis 1 bar.

Vorteilhaft können eine oder mehrere aus der Trennwandkolonne entnommene Hochsiederfraktionen vollständig oder teilweise in die Synthesestufe recycliert werden.

Analog wird vorteilhaft der flüssige, überwiegend Methanol enthaltende Leichtsiederstrom vollständig oder teilweise in die Synthesestufe recycliert.

Die Erfindung wird im Folgenden anhand einer Zeichnung sowie eines Ausführungsbeispiels näher erläutert.

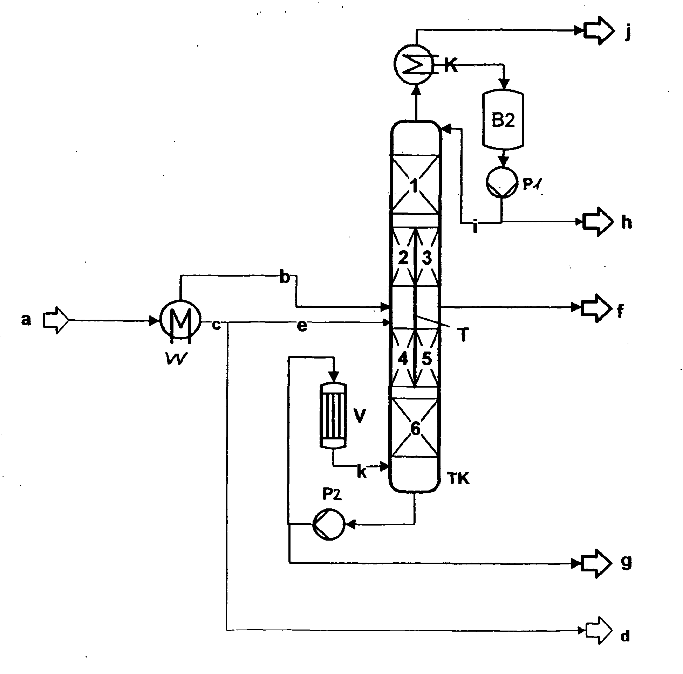

Die einzige Figur 1 zeigt die schematische Darstellung einer Anlage zur destillativen Aufarbeitung des Elektrolyseaustrags zur Herstellung von TMOF durch elektrochemische Oxidation von TME mit Methanol in einer Trennwandkolonne.

Der Elektrolyseaustrag, Strom a, wird in einem Vorverdampfer (VV) teilweise verdampft, wobei ein gasförmiger Strom b erhalten wird, der der Trennwandkolonne (TK) zugeführt wird und ein flüssiger Strom c, der als Strom d teilweise ausgeschleust und vorteilhaft in die Synthesestufe recycliert wird und teilweise als flüssiger Strom e ebenfalls der Trennwandkolonne (TK) zugeführt wird.

Die Trennwandkolonne (TK) ist durch die in Längsrichtung angeordnete Trennwand (T) in die Teilbereiche 1 bis 6 aufgeteilt, d.h. in den oberen gemeinsamen Kolonnenbereich 1, den Zulaufteil mit Verstärkungsteil 2 und Abtriebsteil 4 und den Entnahmeteil mit Verstärkungsteil 5 und Abtriebsteil 3 sowie den unteren gemeinsamen Kolonnenbereich 6.

Der Kopfstrom wird in einem Kondensator (K) im Kolonnenkopf teilweise kondensiert, so teilweise als gasförmige Leichtsiederfraktion j abgezogen und im übrigen in flüssiger Form in einem Behälter B2 gesammelt über eine erste Pumpe (P1), teilweise als Rücklauf i, wieder auf die Kolonne aufgegeben und im übrigen als flüssiger Leichtsiederstrom h abgezogen. Vorteilhaft wird ein weiterer, nicht dargestellter Kondensator nachgeschaltet, dessen Arbeitstemperatur um 10 bis 60 K, bevorzugt 20 bis 40 K unterhalb der Temperatur des Kondensators (K) am Kopf der Trennwandkolonne (TK) liegt. Dadurch kann ein Großteil der noch im Kopfstrom j enthaltenen Leichtsieder als methanolische Lösung niedergeschlagen und somit gegebenenfalls aus dem Prozess ausgeschleust werden.

Der Sumpfstrom wird über eine zweite Pumpe (P2) teilweise als Strom g ausgeschleust, teilweise in einem Sumpfverdampfer, der bevorzugt als Fallfilmapparat ausgebildet ist, teilverdampft und erneut als Strom k in den Sumpf der Trennwandkolonne (TK) zurückgeführt.

Das Wertprodukt TMOF wird als flüssiger Seitenabzug, Strom f, aus dem mittleren Bereich des Entnahmeteils der Trennwandkolonne (TK) abgezogen.

### Ausführungsbeispiel:

TMOF wurde nach dem in der nicht vorveröffentlichten deutschen Patentanmeldung 102 09 195 beschriebenen Ausführungsbeispiel durch elektrochemische Oxidation von TME hergestellt.

Es wurde eine ungeteilte Plattenstapelzelle mit Graphitelektroden in bipolarer Anordnung eingesetzt. Die gesamte Elektrodenfläche betrug 0,145 m² (Anode und Kathode).

In einer kontinuierlich betriebenen Elektrolyse in dieser Zelle erhielt man bei einer Stromdichte von 310 A/m² an Graphitelektroden und einem Zulauf von Methanol zu TME von 1,5 mol zu 1 mol und einem MTBS-Gehalt von 8 Gew.-% im Elektrolyseaustrag bei einem Umsatz von 41 % TME eine Selektivität zu TMOF von 95 % und eine Stromausbeute für TMOF von 78%.

Die Austräge dieser kontinuierlichen Elektrolyse (Gesamtmenge 3181 g, 35 % TMOF, 45 % TME, 10 % Methanol, 8 % MTBS, je 1 % Methylformiat und Methylal) wurden einer Vorverdampfung im Rotationsfilmverdampfer (4,6 dm²) unter Normaldruck bei 150 °C Manteltemperatur und einem Zulauf von 400 g/h zugeführt, mit einer Gesamtmassenbilanz von 100 % und einer TMOF-Bilanz von 99 %. Neben den Schwersiedem TME und MTBS enthielt der Sumpf noch 6 % TMOF. Der Sumpf wurde in die Elektrolyse zurückgeführt. Im Destillat fanden sich 58 % TMOF, 20 % TME, 20% Methanol, 1 % Methylformiat und 1 % Methylal.

Dieses vorverdampfte Gemisch wurde zur weiteren Aufarbeitung einer kontinuierlich betriebenen Labortrennwandkolonne mit einem Innendurchmesser von 50 mm zugeführt, die im Bereich der Trennwand mit einer etwa 1 m hohen Schüttung von 3 mm-Edelstahlgeweberingen, oberhalb der Trennwand mit 50 cm einer Gewebepackung mit einer spezifischen Oberfläche von etwa 950 m²/m³ und unterhalb der Trennwand mit 30 cm derselben Gewebepackung bestückt war.

Die Trennwandkolonne wurde bei einem Kopfdruck von 500 mbar betrieben. Das Verteilungsverhältnis der Flüssigkeit zwischen Zulauf- und Entnahmeteil betrug 1 : 2,5. Die Kolonneninnentemperatur unmittelbar oberhalb der Trennwand wurde über eine temperaturgeführte Rücklaufmengenregelung auf einen Wert um 55 °C eingestellt.

Die Kolonne war mit zwei Kondensatoren ausgestattet, die bei 40 °C beziehungsweise bei -10 °C betrieben wurden. Der Sumpfverdampfer war ein Rotationsfilmverdampfer mit 4,6 dm².

Der Trennwandkolonne wurde das aus der oben beschriebenen Vorverdampfung erhaltene Destillat in einem Mengenstrom von 160 g/h bei Raumtemperatur flüssig in der Mitte des Zulaufbereichs zugeführt. Der Seitenabzug im Entnahmeteil befand sich auf gleicher Höhe mit dem Zulauf. Im Seitenabzug wurde TMOF mit einer Reinheit von 99,75 % gewonnen.

Die Zusammensetzung des Kopfproduktes betrug etwa 94 Gew.-% Methanol und etwa 1 Gew.-% TMOF, Rest Leichtsieder, vor allem Methylformiat und Methylal. Im Nachkondensator wurde eine methanolische Leichtsiederfraktion ausgeschleust, die neben 76 Gew.-% Methanol vor allem die genannten Leichtsieder enthielt.

Aus dem Kolonnensumpf wurde TME in einer Reinheit > 99,7 Gew.-% temperaturgeregelt bei etwa 135 °C abgezogen.

Der Sumpf- sowie der Kopfstrom der Destillation wurden in die elektrochemische Synthesestufe recycliert.

Das Ausführungsbeispiel zeigt somit, dass durch den Einsatz einer einzigen Trennwandkolonne nicht nur das Wertprodukt TMOF in hoher Reinheit, sondern darüber hinaus auch die Edukte Methanol und TME in hoher Reinheit zurückgewonnen wurden.

## Patentansprüche

1. Verfahren zur destillativen Aufarbeitung des Elektrolyseaustrages (a) der elektrochemischen Oxidation von 1,1,2,2-Tetramethoxyethan (TME) mit Methanol zu Trimethylorthoformiat (TMOF) in einem flüssigen Elektrolyten, **dadurch gekennzeichnet, dass** man die destillative Aufarbeitung in einer Trennwandkolonne (TK) mit 30 bis 150 theoretischen Trennstufen durchführt, in der eine Trennwand (T) in Kolonnenlängsrichtung unter Ausbildung eines oberen gemeinsamen Kolonnenbereiches (1), eines unteren gemeinsamen Kolonnenbereiches (6), eines Zulaufteils (2, 4) mit Verstärkungsteil (2) und Abtriebsteil (4) sowie eines Entnahmeteils (3, 5) mit Abtriebsteil (3) und Verstärkungsteil (5) angeordnet ist, mit Zuführung des Elektrolyseaustrags (a) in den mittleren Bereich des Zulaufteils (2, 4), Gewinnung von TMOF als Seitenabzug aus dem mittleren Bereich des Entnahmeteils (3, 5) und Abführung einer oder mehrerer Leichtsiederfraktionen aus dem oberen gemeinsamen Kolonnenbereich (1) und ein oder mehrerer Hochsiederfraktionen aus dem unteren gemeinsamen Kolonnenbereich (6).

2. Verfahren nach Anspruch 1, **dadurch gekennzeichnet, dass** der Elektrolyseaustrag A eine Mischung, enthaltend 25 bis 50 Gew.-% TME, 25 bis 45 Gew.-% TMOF, 5 bis 30 Gew.-% Methanol, 1 bis 20 Gew.-% Methyltributylammoniummethylsulfat (MTBS) sowie je 0,5 bis 2 Gew.-% Methylformiat (MeFO) und Methylal ist.

3. Verfahren nach Anspruch 2, **dadurch gekennzeichnet, dass** die Trennwandkolonne (TK) 60 bis 90 theoretische Trennstufen und einen Betriebsdruck zwischen 300 bis 1500 mbar, bevorzugt zwischen 400 bis 600 mbar, aufweist.

4. Verfahren nach einem der Ansprüche 1 bis 3, **dadurch gekennzeichnet, dass** die theoretischen Trennstufen der Trennwandkolonne (TK) dergestalt auf die einzelnen Kolonnenbereiche 1 bis 6 aufgeteilt sind, dass dieselben jeweils 5 bis 50 %, bevorzugt jeweils 15 bis 30 % der Gesamtzahl der theoretischen Trennstufen der Trennwandkolonne (TK) aufweisen.

5. Verfahren nach einem der Ansprüche 1 bis 3, **dadurch gekennzeichnet, dass** die Summe der Anzahl der theoretischen Trennstufen des Verstärkungsteils (2) und des Abtriebsteils (4) des Zulaufteils (2, 4) 80 bis 110 %, bevorzugt 90 bis 100 % der Summe der Anzahl der theoretischen Trennstufen des Abtriebsteils (3) und Verstärkungsteils (5) des Entnahmeteils (3, 5) beträgt.

6. Verfahren nach einem der Ansprüche 1 bis 5, **dadurch gekennzeichnet, dass** der Elektrolyseaustrag (a) einer ein- oder mehrstufigen Vorverdampfung unterworfen wird und anschließend als gasförmiger Zulauf (b) und/oder flüssiger Zulauf (e) der Trennwandkolonne (TK) im mittleren Bereich des Zulaufteils (2, 4) derselben zugeführt wird.

7. Verfahren nach einem der Ansprüche 1 bis 6, **dadurch gekennzeichnet, dass** eine Leichtsiederfraktion als flüssiger Seitenabzug aus dem oberen gemeinsamen Kolonnenbereich (1) gewonnen wird, bevorzugt von einer theoretischen Trennstufe, die um 3 bis 6 theoretische Trennstufen unterhalb des Kolonnenkopfes liegt.

8. Verfahren nach einem der Ansprüche 1 bis 7, **dadurch gekennzeichnet, dass** aus dem unteren gemeinsamen Kolonnenbereich (6) eine Hochsiederfraktion als flüssiger Seitenabzug gewonnen wird, bevorzugt von einer theoretischen Trennstufe, die um 1 bis 5 theoretische Trennstufen über dem Kolonnensumpf liegt.

9. Verfahren nach einem der Ansprüche 1 bis 8, **dadurch gekennzeichnet, dass** die Zuführung (a, b, e) und der Seitenabzug (f) aus der Trennwandkolonne (TK) auf unterschiedlicher Höhe in der Trennwandkolonne (TK) angeordnet sind, und zwar um 1 bis 20 theoretische Trennstufen, bevorzugt um 5 bis 10 theoretische Trennstufen, wobei der Seitenabzug (f) bevorzugt höher gegenüber der Zuführung (a, b, e) angeordnet ist.

10. Verfahren nach einem der Ansprüche 1 bis 8, **dadurch gekennzeichnet, dass** die Zuführung (a, b, e) und der Seitenabzug (f) aus der Trennwandkolonne (TK) auf gleicher Höhe in der Trennwandkolonne (TK) angeordnet sind.

11. Verfahren nach einem der Ansprüche 1 bis 10, **dadurch gekennzeichnet, dass** einer oder mehrere der Teilbereiche (2, 3, 4, 5) der Trennwandkolonne (TK) mit geordneten Packungen oder Füllkörpern bestückt ist (sind) und/oder, dass die Trennwand (T) in den an einen oder mehreren der Teilbereiche (2, 3, 4, 5) angrenzenden Bereichen wärmeisolierend ausgeführt ist.

12. Verfahren nach einem der Ansprüche 1 bis 11, **dadurch gekennzeichnet, dass** der Seitenabzug (f) flüssig entnommen wird.

13. Verfahren nach einem der Ansprüche 1 bis 12, **dadurch gekennzeichnet, dass** die aus dem oberen gemeinsamen Kolonnenbereich (1) ablaufende Flüssigkeit in einem innerhalb oder außerhalb der Trennwandkolonne (TK) angeordneten Auffangraum gesammelt und durch eine Festeinstellung oder Regelung am oberen Ende der Trennwand (T) so aufgeteilt wird, dass das Verhältnis des Rücklaufstromes im Zulaufteil (2, 4) zum Rücklaufstrom im Entnahmeteil (3, 5) 0,1 bis 2,5, bevorzugt 0,4 bis 0,8, bei überwiegend flüssiger Zuführung (a, b, e) und bevorzugt 1,0 bis 2,0 bei gasförmiger Zuführung (b) beträgt.

14. Verfahren nach einem der Ansprüche 1 bis 13, **dadurch gekennzeichnet, dass** die Trennwandkolonne (TK) am oberen und/oder am unteren Ende der Trennwand (T) Probeentnahmemöglichkeiten aufweist, über die aus der Kolonne kontinuierlich oder in zeitlichen Abständen flüssige und/oder gasförmige Proben entnommen und hinsichtlich ihrer Zusammensetzung, bevorzugt gaschromatographisch, untersucht werden.

15. Verfahren nach einem der Ansprüche 1 bis 14, **dadurch gekennzeichnet, dass** die Entnahme des Seitenabzugs (f) standgeregelt erfolgt und als Regelgröße der Flüssigkeitsstand im Sumpf der Trennwandkolonne (TK) verwendet wird.

16. Verfahren nach einem der Ansprüche 1 bis 15, **dadurch gekennzeichnet, dass** die Flüssigkeitsverteilung in den einzelnen Teilbereichen (2, 3, 4, 5) der Kolonne gezielt ungleichmäßig eingestellt wird, bevorzugt dass die Flüssigkeit in den Teilbereichen (2, 5) verstärkt im Wandbereich und in den Teilbereichen (3, 4) reduziert im Wandbereich aufgegeben wird.

17. Verfahren nach einem der Ansprüche 1 bis 16, **dadurch gekennzeichnet, dass** die Position der Trennwand (T) in den einzelnen Teilbereichen (2, 3, 4, 5) der Trennwandkolonne (TK) so angepasst werden kann, dass die Querschnittsflächen von Zulaufteil (2, 4) und Entnahmeteil (3, 5) unterschiedlich sind.

18. Verfahren nach einem der Ansprüche 1 bis 17, **dadurch gekennzeichnet, dass** anstelle einer Trennwandkolonne (TK) thermisch gekoppelte Kolonnen eingesetzt werden.

## Claims

1. A process for the distillative working-up of the electrolysis discharge (a) of the electrochemical oxidation of 1,1,2,2-tetramethoxyethane (TME) with methanol to give trimethyl orthoformate (TMOF) in a liquid electrolyte, wherein the distillative working-up is carried out in a dividing wall column (TK) having from 30 to 150 theoretical plates, in which a dividing wall (T) is arranged in the longitudinal direction of the column with formation of an upper common column region (1), of a lower common column region (6), of a feed section (2, 4) with rectification section (2) and stripping section (4) and of a take-off section (3, 5) with stripping section (3) and rectification section (5), with feeding of the electrolysis discharge (a) into the middle region of the feed section (2, 4), recovery of TMOF as a side take-off from the middle region of the take-off section (3, 5) and removal of one or more low boiler fractions from the upper common column region (1) and one or more high boiler fractions from the lower common column region (6).

2. The process according to claim 1, wherein the electrolysis discharge A is a mixture comprising from 25 to 50% by weight of TME, from 25 to 45% by weight of TMOF, from 5 to 30% by weight of methanol, from 1 to 20% by weight of methyltributylammonium methylsulfate (MTBS) and from 0.5 to 2% by weight each of methyl formate (MeFO) and methylal.

3. The process according to claim 2, wherein the dividing wall column (TK) has from 60 to 90 theoretical plates and an operating pressure of from 300 to 1 500, preferably from 400 to 600, mbar.

4. The process according to any of claims 1 to 3, wherein the theoretical plates of the dividing wall column (TK) are distributed over the individual column regions 1 to 6 in such a way that these each have from 5 to 50, preferably each from 15 to 30, % of the total number of theoretical plates of the dividing wall column (TK).

5. The process according to any of claims 1 to 3, wherein the sum of the number of theoretical plates of the rectification section (2) and of the stripping section (4) of the feed section (2, 4) is from 80 to 110, preferably from 90 to 100, % of the sum of the number of theoretical plates of the stripping section (3) and of the rectification section (5) of the take-off section (3, 5).

6. The process according to any of claims 1 to 5, wherein the electrolysis discharge (a) is subjected to a one-stage or multistage preliminary evaporation and then fed as gaseous feed (b) and/or liquid feed (e) in the middle region of the feed section (2, 4) of the dividing wall column (TK).

7. The process according to any of claims 1 to 6, wherein a low boiler fraction is obtained as a liquid side take-off from the upper common column region (1), preferably from a theoretical plate which is from 3 to 6 theoretical plates below the top of the column.

8. The process according to any of claims 1 to 7, wherein a high boiler fraction is obtained as a liquid side take-off from the lower common column region (6), preferably from a theoretical plate which is from 1 to 5 theoretical plates above the bottom of the column.

9. The process according to any of claims 1 to 8, wherein the feed (a, b, e) and the side take-off (f) from the dividing wall column (TK) are arranged at different heights in the dividing wall column (TK), in particular differing by from 1 to 20, preferably from 5 to 10, theoretical plates, the side take-off (f) preferably being arranged higher than the feed (a, b, e).

10. The process according to any of claims 1 to 8, wherein the feed (a, b, e) and the side take-off (f) from the dividing wall column (TK) are arranged at the same height in the dividing wall column (TK).

11. The process according to any of claims 1 to 10, wherein one or more of the part-regions (2, 3, 4, 5) of the dividing wall column (TK) is or are loaded with stacked packings or dumped packings and/or wherein the dividing wall (T) is designed to be heat-insulated in the regions adjacent to one or more of the part-regions (2, 3, 4,5)

12. The process according to any of claims 1 to 11, wherein the side take-off (f) is removed in liquid form.

13. The process according to any of claims 1 to 12, wherein the liquid flowing out of the upper common column region (1) is collected in a collecting space arranged inside or outside the dividing wall column (TK) and, by means of a fixed setting or regulation at the upper end of the dividing wall (T), is divided in such a way that the ratio of the reflux stream in the feed section (2, 4) to the reflux stream in the take-off section (3, 5) is from 0.1 to 2.5, preferably from 0.4 to 0.8, in the case of a predominantly liquid feed (a, b, e) and preferably from 1.0 to 2.0 in the case of a gaseous feed (b).

14. The process according to any of claims 1 to 13, wherein the dividing wall column (TK) has, at the upper and/or at the lower end of the dividing wall (T), sampling facilities via which liquid and/or gaseous samples are taken from the column continuously or at time intervals and are investigated with regard to their composition, preferably by gas chromatography.

15. The process according to any of claims 1 to 14, wherein the side take-off (f) is removed with level regulation, and the liquid level in the bottom of the dividing wall column (TK) is used as a control variable.

16. The process according to any of claims 1 to 15, wherein the liquid distribution is made nonuniform in a specific manner in the individual part-regions (2, 3, 4, 5) of the column, preferably wherein the liquid is fed in to a greater extent in the wall region in the part-regions (2, 5) and to a reduced extent in the wall region in the part-regions (3, 4).

17. The process according to any of claims 1 to 16, wherein the position of the dividing wall (T) in the individual part-regions (2, 3, 4, 5) of the dividing wall column (TK) can be adapted so that the cross-sectional areas of feed section (2, 4) and take-off section (3, 5) are different.

18. The process according to any of claims 1 to 17, wherein thermally coupled columns are used instead of a dividing wall column (TK).

## Revendications

1. Procédé de traitement par distillation de la sortie d'électrolyse (a) de l'oxydation électrochimique de 1,1,2,2-tétraméthoxyéthane (TME) avec du méthanol en triméthylorthoformiate (TMOF) dans un électrolyte liquide, **caractérisé en ce qu'**on exécute le traitement par distillation dans une colonne de distillation à paroi de séparation (TK) avec 30 à 150 plateaux de séparation théoriques, dans laquelle une paroi de séparation (T) est agencée dans le sens longitudinal de colonne avec formation d'une zone commune supérieure de colonne (1), d'une zone commune inférieure de colonne (6), d'une partie d'écoulement (2, 4) avec partie de concentration (2) et partie d'entraînement (4) ainsi que d'une partie de prélèvement (3, 5) avec partie d'entraînement (3) et partie de concentration (5), avec amenée de la sortie d'électrolyte (a) dans la zone centrale de la partie d'écoulement (2, 4), obtention de TMOF comme sous-tirage latéral d'une zone centrale de la partie de prélèvement (3, 5) et évacuation d'une ou de plusieurs fractions de produits à bas point d'ébullition de la zone commune supérieure de colonne (1) et d'une ou de plusieurs fractions de produits à point d'ébullition élevé de la zone commune inférieure de colonne (6).

2. Procédé selon la revendication 1, **caractérisé en ce que** la sortie d'électrolyse A est un mélange contenant 25 à 50% en poids de TME, 25 à 45% en poids de TMOF, 5 à 30% en poids de méthanol, 1 à 20% en poids de méthylsulfate de méthyltributylammonium (MTBS) ainsi que chaque fois 0,5 à 2% en poids de méthylformiate (MeFO) et de méthylal.

3. Procédé selon la revendication 2, **caractérisé en ce que** la colonne de distillation à paroi de séparation (TK) présente 60 à 90 plateaux de séparation théoriques et une pression de fonctionnement entre 300 et 1.500 mbars, de préférence entre 400 et 600 mbars.

4. Procédé selon l'une quelconque des revendications 1 à 3, **caractérisé en ce que** les plateaux de séparation théoriques de la colonne de distillation à paroi de séparation (TK) sont divisés entre les différentes zones de colonne 1 à 6 de telle façon que celles-ci présentent chaque fois 5 à 50%, de préférence chaque fois 15 à 30% du nombre total des plateaux de séparation théoriques de la colonne de distillation à paroi de séparation (TK).

5. Procédé selon l'une quelconque des revendications 1 à 3, **caractérisé en ce que** la somme du nombre des plateaux de séparation théoriques de la partie de concentration (2) et de la partie d'entraînement (4) de la partie d'écoulement (2, 4) est de 80 à 110%, de préférence de 90 à 100% de la somme du nombre des plateaux de séparation théoriques de la partie d'entraînement (3) et de la partie de concentration (5) de la partie de prélèvement (3, 5).

6. Procédé selon l'une quelconque des revendications 1 à 5, **caractérisé en ce que** la sortie d'électrolyse (a) est soumise à une évaporation préalable en une ou plusieurs étapes, puis est conduite en écoulement gazeux (b) et/ou écoulement liquide (e) à la colonne de distillation à paroi de séparation (TK) dans la zone centrale de la partie d'écoulement (2, 4) de celle-ci.

7. Procédé selon l'une quelconque des revendications 1 à 6, **caractérisé en ce qu'**une fraction de produits à bas point d'ébullition est obtenue comme sous-tirage latéral liquide de la zone commune supérieure de colonne (1), de préférence d'un plateau de séparation théorique situé sur 3 à 6 plateaux de séparation théoriques en dessous de la tête de colonne.

8. Procédé selon l'une quelconque des revendications 1 à 7, **caractérisé en ce qu'**une fraction de produits à haut point d'ébullition est obtenue de la zone commune inférieure de colonne (6) sous forme d'un sous-tirage latéral liquide, de préférence d'un plateau de séparation théorique situé sur 1 à 5 plateaux de séparation théoriques au-dessus du bas de colonne.

9. Procédé selon l'une quelconque des revendications 1 à 8, **caractérisé en ce que** l'amenée (a, b, e) et le sous-tirage latéral (f) de la colonne de distillation à paroi de séparation (TK) sont agencés à une hauteur différente dans la colonne de distillation à paroi de séparation (TK) c'est-à-dire sont agencés sur 1 à 20 plateaux de séparation théoriques, de préférence de 5 à 10 plateaux de séparation théoriques, le sous-tirage latéral (f) étant agencé de préférence plus haut par rapport à l'amenée (a, b, e).

10. Procédé selon l'une des revendications 1 à 8, **caractérisé en ce que** l'amenée (a,b,e) et le sous-tirage latéral (f) de la colonne de distillation à paroi de séparation (TK) sont agencés à une hauteur identique dans la colonne de distillation à paroi de séparation (TK).

11. Procédé selon l'une des revendications 1 à 10, **caractérisé en ce qu'**une ou plusieurs des zones partielles (2, 3, 4, 5) de la colonne de distillation à paroi de séparation (TK) est ou sont équipées de paquets ordonnés ou de corps de charge et/ou **en ce que** la paroi de séparation (T) est réalisée de manière thermiquement isolée dans les zones jouxtant une ou plusieurs des zones partielles (2, 3, 4, 5).

12. Procédé selon l'une quelconque des revendications 1 à 11, **caractérisé en ce que** le sous-tirage latéral (f) est prélevé liquide.

13. Procédé selon l'une quelconque des revendications 1 à 12, **caractérisé en ce que** le liquide s'écoulant de la zone commune supérieure de colonne (1) est recueilli dans une chambre de captage agencée à l'intérieur ou à l'extérieur de la colonne de distillation à paroi de séparation (TK) et est divisé par un réglage des solides ou un réglage à l'extrémité supérieure de la paroi de séparation (T) de telle sorte que le rapport du courant de reflux dans la partie d'écoulement (2, 4) au courant de reflux dans la partie de prélèvement (3, 5) est de 0,1 à 2,5, de préférence 0,4 à 0,8 lors d'une amenée principalement liquide (a, b, e) et de préférence de 1,0 à 2,0 lors d'une amenée gazeuse (b).

14. Procédé selon l'une des revendications 1 à 13, **caractérisé en ce que** la colonne de distillation à paroi de séparation (TK) présente sur l'extrémité supérieure et/ou sur l'extrémité inférieure de la paroi de séparation (T) des accessoires de prélèvement d'échantillons par lesquels des échantillons liquides et/ou gazeux sont prélevés de la colonne en continu ou à intervalles temporels, et sur le plan de leur composition, sont examinés par chromatographie en phase gazeuse.

15. Procédé selon l'une quelconque des revendications 1 à 14, **caractérisé en ce que** le prélèvement du sous-tirage latéral (f) s'effectue en réglant le niveau et qu'on utilise comme grandeur de réglage le niveau de liquide au bas de la colonne de distillation à paroi de séparation (TK).

16. Procédé selon l'une quelconque des revendications 1 à 15, **caractérisé en ce que** la répartition du liquide dans les différentes zones partielles (2, 3, 4, 5) de la colonne est réglée pour être expressément irrégulière, de préférence en ce que, dans les zones partielles (2, 5), plus de liquide est réparti dans la zone de paroi et, dans les zones partielles (3, 4), moins de liquide est réparti dans la zone de paroi.

17. Procédé selon l'une quelconque des revendications 1 à 16, **caractérisé en ce que** la position de la paroi de séparation (T) dans les différentes zones partielles (2, 3, 4, 5) de la colonne de distillation à paroi de séparation peut être adaptée de telle façon que les superficies de la section de la partie d'écoulement (2, 4) et de la partie de prélèvement (3, 5) sont différentes.

18. Procédé selon l'une quelconque des revendications 1 à 17, **caractérisé en ce qu'**au lieu d'une colonne de distillation à paroi de séparation (TK), on utilise des colonnes thermiquement couplées.
